(19) Europäisches Patentamt
European Patent Office
Office européen des brevets

(11) Numéro de publication: **0 172 097 B1**

# (12) FASCICULE DE BREVET EUROPEEN

(45) Date de publication du fascicule du brevet: **01.03.89**

(51) Int. Cl.[4]: **C 07 D 471/04**, A 61 K 31/435 // (C07D471/04, 235:00, 221:00)

(21) Numéro de dépôt: **85401555.9**

(22) Date de dépôt: **30.07.85**

(54) **Imidazo(1,2-a)quinolines, leur préparation et leur application en thérapeutique.**

(30) Priorité: **07.08.84 FR 8412446**

(43) Date de publication de la demande: **19.02.86 Bulletin 86/8**

(45) Mention de la délivrance du brevet: **01.03.89 Bulletin 89/9**

(84) Etats contractants désignés: **AT BE CH DE FR GB IT LI LU NL SE**

(56) Documents cités: **EP-A- 0 050 563**

(73) Titulaire: **SYNTHELABO, 58, rue de la Glacière, F-75621 Paris Cedex 13 (FR)**

(72) Inventeur: **George, pascal, 39, rue Henri de Vilmorin, F-92440 Vitry s/Seine (FR)**
Inventeur: **De Peretti, Danielle, 14, rue Ampere, F-92160 Antony (FR)**

(74) Mandataire: **Ludwig, Jacques et al, SYNTHELABO Service Brevets 58, rue de la Glacière, F-75621 Paris Cedex 13 (FR)**

**EP 0 172 097 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés d'imidazo [1,2-a]quinolines de formule générale I, figurant dans le schéma de la page suivante, formule dans laquelle

X représente l'hydrogène, un halogène ou un groupe méthyle, méthoxy, méthylthio ou méthylsulfonyle,

Y représente l'hydrogène, un halogène ou un groupe méthyle en position 6, 7 ou 8, et

R1 et R2, pris séparément, représentent chacun l'hydrogène ou un groupe méthyle ou éthyle, ou bien

R1 ou R2 forment ensemble une chaîne tétraméthylène, pentaméthylène, méthyl-3 aza-3 pentaméthylène, éthoxycarbonyl-3 aza-3 pentaméthylène ou oxa-3 pentaméthylène.

Les sels d'addition que peuvent former ces composés avec des acides font également partie de l'invention.

Des dérivés d'imidazo[1,2-a]pyridines, homologues des imidazo[1,2-a]quinolines de l'invention, sont décrits dans le EP-A-0 050 563. Outre leur différence de structure chimique, les composés selon l'invention présentent aussi une différence de profil pharmacologique.

Les composés de l'invention peuvent être préparés selon le schéma de la page suivante.

On soumet d'abord la quinoline de formule II ou l'amino-2 quinoline de formule IV à l'action d'une α-bromo-acétophénone portant le substituant X défini ci-dessus.

Dans le cas de la quinoline (II), la réaction a lieu à chaud dans un solvant tel que le chlorure de méthylène ou le dichloro-1,2 éthane. On obtient un composé ionique de formule III que l'on cyclise à chaud en présence d'acétate d'ammonium et de chlorure ferrique pour obtenir le composé de formule V.

Dans le cas de l'amino-2 quinoline (IV), on obtient directement le composé de formule V en

Schéma

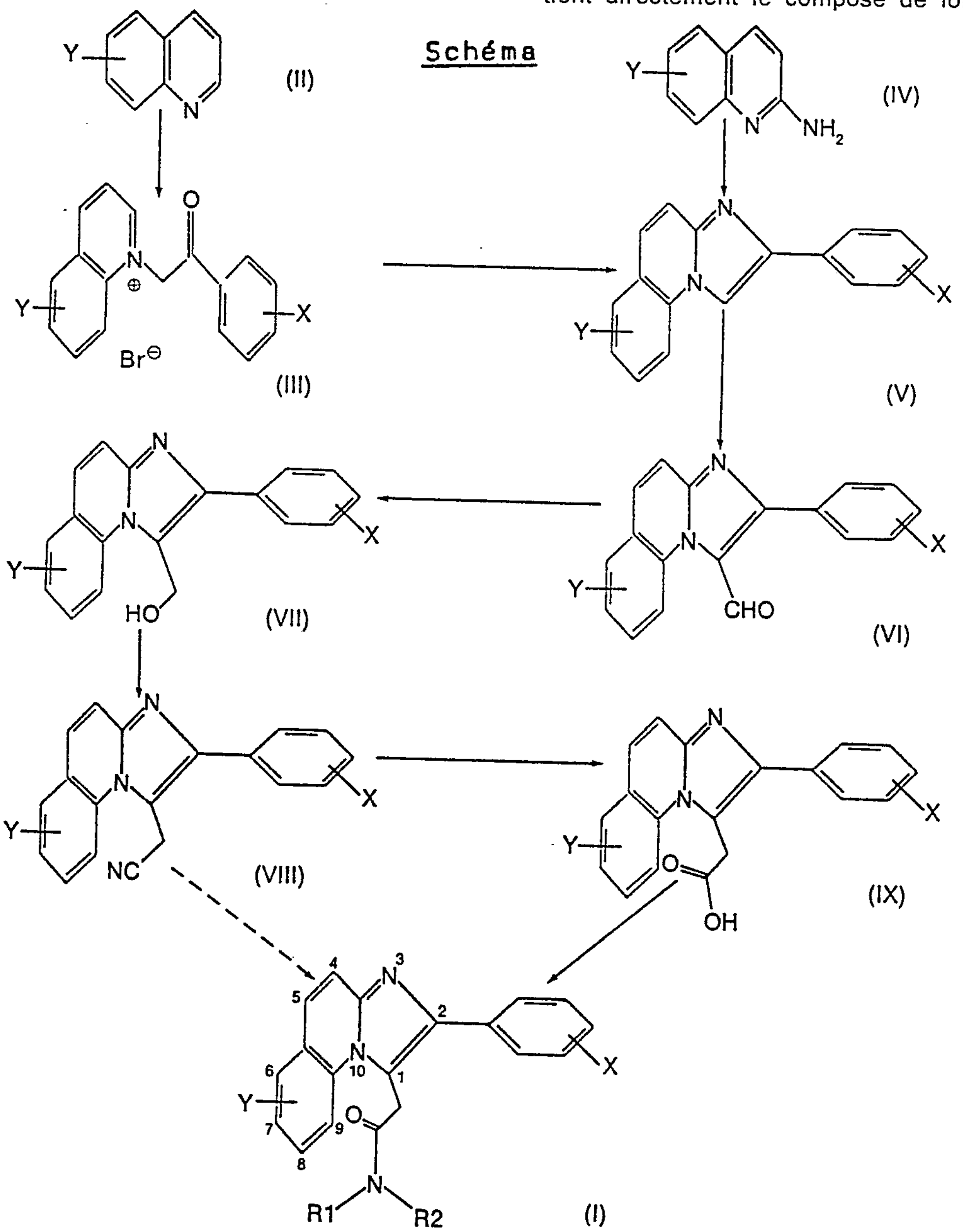

(II) (IV) (III) (V) (VII) (VI) (VIII) (IX) (I)

effectuant la réaction en présence d'une base et dans un solvant alcoolique.

On effectue ensuite une formylation du composé (V) ainsi obtenu, par exemple au moyen du réactif que l'on obtient par action du chlorure d'oxalyle sur le diméthylformamide, puis hydrolyse de cet adduit.

On réduit ensuite l'aldéhyde (VI) ainsi obtenu en un alcool de formule VII, de manière connue, par exemple par action du borohydrure de sodium ou de potassium.

Puis on soumet l'alcool (VII) d'abord à une tosylation, par exemple au moyen de chlorure de paratoluène sulfonyle, en présence de pyridine, et ensuite à l'action du cyanure de sodium ou de potassium en milieu aqueux, obtenant ainsi le nitrile de formule VIII.

On hydrolyse ensuite ce nitrile en acide carboxylique de manière connue, par exemple à chaud et en milieu acide chlorhydrique, pour obtenir le composé de formule IX.

On en prépare finalement l'amide correspondant en soumettant l'acide (IX) d'abord à l'action du carbonyldiimidazole, puis à l'action d'une amine de formule R1-NH-R2 dans laquelle R1 et R2 sont tels que définis ci-dessus.

Dans le cas de l'amide non substitué de formule I (R1=R2=H), on peut obtenir directement le composé final par hydrolyse partielle du nitrile (VIII) en milieu basique aqueux.

Enfin il va de soi que l'on peut aussi obtenir les amides N,N-diméthylés par méthylation de l'amide non substitué (R1=R2=H) au moyen de iodométhane en présence d'hydrure de sodium.

Les exemples A et B ci-dessous illustrent les deux variantes de la préparation du composé de formule V.

Les exemples suivants illustrent la préparation de quelques composés selon l'invention.

Les microanalyses et les spectres IR et RMN confirment leur structure.

Exemple A.

(Chloro-4 phényl)-2 imidazo[1,2-a]quinoline.

On mélange 17,7 g (0,123 mole) d'amino-2 quinoline, 28,7 g (1 équivalent) de α-bromo-p-chloroacétophénone et 20,6 g (2 équivalents) de bicarbonate de sodium dans 180 ml de n-propanol et on chauffe au reflux pendant 20 heures. On filtre le précipité obtenu, le lave à l'éthanol, à l'eau et on le sèche. On obtient un solide blanc que l'on recristallise dans le nitrométhane.

F = 206–208 °C.

Exemple B.

(Chloro-4 phényl)-2 imidazo[1,2-a]quinoline.

On mélange 23,3 g (0,1 mole) de α-bromo-p-chloro acétophénone et 13 ml (1,1 équivalent) de quinoline avec 100 ml de chlorure de méthylène. On chauffe au reflux 1 heure puis ajoute au mélange réactionnel 100 ml d'éther et on refroidit. On filtre et sèche le précipité jaune obtenu.

Dans un autoclave de 1 litre on mélange 38,3 g (0,105 mole) de l'ammonium quaternaire précédent, 48,6 g (0,63 mole) d'acétate d'ammonium, 55 g (0,34 mole) de chlorure ferrique dans 270 ml d'acide acétique, et on chauffe à 140 °C pendant 13 heures. On filtre le solide obtenu, le lave à l'acide acétique puis à l'eau; on le sèche, le recristallise dans l'éthanol puis dans le nitrométhane.

Exemple 1.

(Chloro-4 phényl)-2 imidazo[1,2-a]quinoline-1-acétamide.

a) (Chloro-4 phényl)-2 imidazo[1,2-a]quinoline-1-carboxaldéhyde.

On refroidit à −20 °C 150 ml de diméthylformamide sec et on y ajoute goutte à goutte 17,4 ml (0,2 mole) de chlorure d'oxalyle. On ajoute ensuite 13,6 g (0,05 mole) du composé obtenu selon l'exemple A ou B et on agite 15 heures à température ambiante et 15 heures à 55 °C.

Puis on verse le mélange dans 1 litre d'eau, on filtre, on alcalinise le précipité, on le lave à l'eau jusqu'à pH neutre et on le sèche. On le recristallise dans le nitrométhane.

F = 199–200 °C.

b) Hydroxyméthyl-1 (chloro-4 phényl)-2 imidazo[1,2-a]quinoline.

On met 13,3 g (0,043 mole) de l'aldéhyde précédent en suspension dans 300 ml d'éthanol absolu sec.

On y ajoute 825 mg (0,5 équivalent) de borohydrure de sodium et on agite 18 heures à température ambiante. Puis on évapore à sec, on reprend le résidu à l'eau, on ajuste le pH à 8 par de l'acide chlorhydrique dilué, on filtre, on lave le précipité à l'eau, à l'acétone, puis à l'éther, et on le sèche.

F = 236–237 °C (décomposition).

c) Cyanométhyl-1 (chloro-4 phényl)-2 imidazo[1,2-a] quinoline.

On mélange 7,8 g (0,025 mole) de l'alcool précédent et 5,3 g (1,1 équivalent) de chlorure de p-toluènesulfonyle dans 100 ml de pyridine. On chauffe le mélange réactionnel à 40 °C pendant 8 heures, puis on l'agite à température ambiante pendant 60 heures. On le reprend ensuite avec 700 ml d'eau et 300 ml de chlorure de méthylène, on filtre le précipité entre les deux phases et on le verse dans 400 ml d'eau contenant 3,7 g (0,075 mole) de cyanure de sodium et 2,1 g (0,025 mole) de bicarbonate de sodium. On chauffe le mélange au reflux pendant 15 heures puis on le refroidit et on le filtre. On lave le précipité à l'eau, on l'extrait avec du chlorure de méthylène et on sèche l'extrait sur sulfate de sodium. On purifie le nitrile par chromatographie sur gel de silice.

Après cristallisation dans l'éther et séchage on obtient un solide blanc.

F = 221–223 °C.

d) (Chloro-4 phényl)-2 imidazo[1,2-a]quinoline-1-acétamide.

On mélange 7 g (0,022 mole) du nitrile précédent et 6 g (5 équivalents) de potasse dans 200 ml d'éthanol et 50 ml d'eau, et on chauffe 3 heures au reflux. Puis on évapore à sec, on reprend le résidu à l'eau, on le filtre, on le lave à l'eau jusqu'à pH neutre et on le sèche.
F = 298–300 °C.

Exemple 2.
(Chloro-4 phényl)-2 N-méthyl imidazo[1,2-a]quinoline-1-acétamide.

a) Acide (chloro-4 phényl)-2 imidazo[1,2-a]quinoline-1-acétique.

On verse 3,5 g (0,011 mole) du nitrile obtenu selon l'exemple 1c) dans un mélange de 10 ml d'acide chlorhydrique concentré et de 20 ml d'acide acétique. On porte ce mélange réactionnel au reflux pendant 8 heures, puis on ajoute encore 10 ml d'acide chlorhydrique concentré et rechauffe au reflux pendant 8 heures. Puis on évapore à sec, on reprend le résidu à l'eau et on filtre le précipité. On remet le précipité en suspension dans l'eau, on ajuste le pH à 5 par de la soude diluée, on le filtre, on le lave à l'éthanol, à l'acétone et à l'éther et on le sèche.
F = 246–248 °C (décomposition).

b) (Chloro-4 phényl)-2 N-méthyl imidazo[1,2-a]quinoline-1-acétamide.

On met 2,5 g (0,0074 mole) de l'acide précédent en suspension dans 50 ml de tétrahydrofuranne sec, on ajoute 1,4 g (1,2 équivalent) de carbonyldiimidazole et on chauffe le mélange vers 40 °C pendant 2 heures.

Puis on y fait barboter de la méthylamine pendant 30 minutes, et on continue l'agitation pendant 15 heures. On évapore le mélange à sec, on reprend le résidu avec de l'eau et du chlorure de méthylène, on sépare la phase organique, on la lave à l'eau, on la sèche et on l'évapore. On purifie le résidu par chromatographie et on le laisse cristalliser dans l'éther.
F = 289–292 °C (décomposition).

Exemple 3.
(Chloro-4 phényl)-2 N,N-diméthyl imidazo[1,2-a]quinoline-1-acétamide.

A une suspension de 3,35 g (0,01 mole) du composé obtenu selon l'exemple 1d) dans 200 ml de tétrahydrofuranne on ajoute 1,05 g (0,022 mole) d'hydrure de sodium à 50% dans l'huile, 0,1 ml de diméthylformamide puis rapidement 1,9 ml (3 équivalents) de iodométhane. On agite 15 heures sous argon à température ambiante puis on évapore à sec, on reprend le résidu avec de l'eau et du chlorure de méthylène, on lave à l'eau la phase organique, on la sèche et on l'évapore. On purifie le résidu par chromatographie.
F = 190–191,5 °C.

Exemple 4.
(Chloro-4 phényl)-2 N,N-tétraméthylène imidazo[1,2-a]quinoline-1-acétamide.

On met 2,5 g (0,0074 mole) de l'acide obtenu selon l'exemple 2a) en suspension dans 50 ml de tétrahydrofuranne sec, on ajoute 1,4 g (1,2 équivalent) de carbonyldiimidazole et on chauffe le mélange vers 40 °C pendant 2 heures.

Puis on ajoute 0,63 g (1,2 équivalent) de pyrrolidine et on continue d'agiter à température ambiante pendant 15 heures. On évapore le mélange à sec, on reprend le résidu avec de l'eau et du chlorure de méthylène, on sépare la phase organique, on la lave à l'eau, on la sèche et on l'évapore. On purifie le résidu par chromatographie et on le laisse cristalliser dans l'éther.
F = 217–218 °C.

Le tableau ci-après illustre les structures et propriétés physiques des composés de l'invention.

Tableau

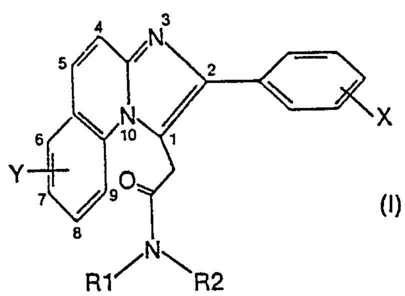

(I)

| Composé | Y | X | $R_1$ | $R_2$ | Base/sel(*) | F(°C |
|---|---|---|---|---|---|---|
| 1 (Ex. 1) | H | 4–Cl | H | H | 00 | 298–300 |
| 2 (Ex. 2) | H | 4–Cl | H | $CH_3$ | 00 | 289–292 |
| 3 (Ex. 3) | H | 4–Cl | $CH_3$ | $CH_3$ | 00 | 190–191,5 |
| 4 (EX. 4) | H | 4–Cl | $-(CH_2)_4-$ | | 00 | 217–218 |
| 5 | H | 4–Cl | $-(CH_2)_5-$ | | 00 | 163–164 |
| 6 | H | 4–Cl | $-(CH_2)_2-N(CH_3)-(CH_2)_2-$ | | 15 | 162–165 |

(*): 00 = base libre
15 = méthanesulfonate

Tableau (suite)

| Composé | Y | X | $R_1$ | $R_2$ | Base/sel(*) | F(°C |
|---|---|---|---|---|---|---|
| 7 | H | 4–Cl | $-(CH_2)_2-N(COOC_2H_5)-(CH_2)_2-$ | | 00 | 188–190 |
| 8 | H | 4–Cl | $-(CH_2)_2-O-(CH_2)_2-$ | | 00 | 228–232 |
| 9 | H | 4–Cl | H | $C_2H_5$ | 00 | 268–270 |
| 10 | 8–Cl | 4–Cl | H | $CH_3$ | 00 | 290,5–291 |
| 11 | 8–Cl | 4–Cl | $CH_3$ | $CH_3$ | 00 | 259–260 |
| 12 | 7–Cl | 4–Cl | H | $CH_3$ | 00 | 300–302 |
| 13 | 7–Cl | 4–Cl | $CH_3$ | $CH_3$ | 00 | 248–250 |
| 14 | 6–Cl | 4–Cl | H | $CH_3$ | 00 | 306–307 |
| 15 | 6–Cl | 4–Cl | $CH_3$ | $CH_3$ | 00 | 248–249 |
| 16 | 7–F | 4–Cl | H | $CH_3$ | 00 | 304–305 |
| 17 | 7–$CH_3$ | 4–Cl | H | $CH_3$ | 00 | 269–271 |
| 18 | H | 4–$CH_3$ | H | $CH_3$ | 00 | 265–266 |
| 19 | H | 4–$CH_3$ | $CH_3$ | $CH_3$ | 00 | 181–182,5 |
| 20 | H | 4–$OCH_3$ | H | $CH_3$ | 00 | 264–265 |
| 21 | H | 4–$OCH_3$ | $CH_3$ | $CH_3$ | 00 | 194–196 |
| 22 | H | 4–$SCH_3$ | H | $CH_3$ | 00 | 284–287 |
| 23 | H | 4–$SCH_3$ | $CH_3$ | $CH_3$ | 00 | 172–173 |
| 24 | H | 3–Cl | H | $CH_3$ | 00 | 261–262 |
| 25 | H | 3–Cl | $CH_3$ | $CH_3$ | 00 | 138–140 |
| 26 | H | 2–Cl | H | $CH_3$ | 00 | 222–224 |
| 27 | H | 2–Cl | $CH_3$ | $CH_3$ | 00 | 165–167 |
| 28 | H | H | H | $CH_3$ | 00 | 264–266 |
| 29 | H | H | $CH_3$ | $CH_3$ | 00 | 173–175 |
| 30 | H | 4–$SO_2CH_3$ | H | $CH_3$ | 00 | 279–280 |
| 31 | H | 4–$SO_2CH_3$ | $CH_3$ | $CH_3$ | 00 | 244–245 |

(*): 00 = base libre
15 = méthanesulfonate

Les composés de l'invention ont fait l'objet d'essais pharmacologiques qui ont mis en évidence leur intérêt comme substances à activités thérapeutiques.

Antagonisme vis-à-vis des convulsions cloniques induites par le Cardiazol(R) chez la souris.

L'essai est inspiré du protocole décrit par Goodman et al., J. Pharm. Exp. Ther., 108, 168–176. Les souris reçoivent les produits à tester, ou le solvant seul, 30 minutes (voie i.p.) ou 60 minutes (voie orale) avant l'injection de 35 mg/kg de Cardiazol(R) par voie intraveineuse. Les animaux sont ensuite observés pendant une heure et, pour chaque lot, le pourcentage de souris présentant des convulsions cloniques est noté (100% de convulsions cloniques et 10 à 20% de convulsions toniques chez les animaux de contrôle). Pour chaque dose, on calcule le pourcentage de protection par rapport aux animaux de contrôle, ce qui permet de déterminer graphiquement la $DA_{50}$, dose qui protège 50% des animaux vis-à-vis des effets convulsivants du Cardiazol(R). Les $DA_{50}$ des composés de l'invention se situent entre 0,1 et 30 mg/kg pour la voie intrapéritonéale et entre 0,1 et 30 mg/kg pour la voie orale.

Test «d'enfouissement» chez la souris («Burying test»).

Ce test est inspiré de la méthode décrite par Pinel J.P.J., Treit D., Ladak F. et MacLennan A.J. dans Animal learning and behavior, 8, 447–451 (1980).

La présence de corps étrangers dans l'environnement habituel d'un animal constitue une situation aversive à laquelle l'animal réagit en enfouissant l'objet de l'agression (billes de verre) dans la sciure de sa cage.

Les anxiolytiques ont pour effet de diminuer l'appréhension causée par la présence étrangère: les animaux enfouissent moins. On compte alors le nombre de billes restées non enfouies.

Les produits à étudier sont administrés à des souris mâles de souche CD1 (Charles River) 30 minutes (voie intrapéritonéale) ou 60 minutes (voie orale) avant que ces dernières soient placées dans des cages contenant 25 billes de verre. Au bout de 30 minutes on compte le nombre de billes restées non enfouies. Un pourcentage est calculé entre les animaux traités et les animaux témoins.

On détermine ainsi la $DA_{50}$, dose active 50%, qui est la dose de composé (en mg/kg) diminuant de moitié le nombre de billes enfouies, en comparaison avec les animaux témoins. Les $DA_{50}$ des composés de l'invention se situent entre 0,3 et 30 mg/kg par voie intrapéritonéale.

Test de conflit de la boisson chez le rat.

Ce test est décrit par Vogel J.R., Beer B. et Clody D.E. dans Psychopharmacologia, 21, 1–7 (1971).

Des rats mâles Wistar (IFFA Credo) sont utilisés. L'eau de boisson leur est retirée 24 h avant le test. Le jour du test, 30 minutes après traitement par voie intrapéritonéale avec les composés de l'invention, chaque rat est placé dans une cage en matière plastique transparente (24 × 20 × 21 cm) à plancher grillagé électrifiable. De l'eau de boisson est distribuée par l'intermédiaire d'une pipette sortant de 2 cm d'une paroi de la cage et placée à 3 cm au-dessus du plancher de la cage.

Après une exploration de 10 à 90 secondes, l'animal trouve la pipette et commence à boire. Après avoir donné 20 coups de langue (enregistrés par un anxiomètre OMNITECH), le rat reçoit, au niveau de la langue un choc électrique de 0,07 mA (délivré par l'anxiomètre) qui s'arrête lorsque le rat quitte la pipette. Une session de 3 minutes commence après un premier choc, l'animal continuant de recevoir un choc tous les 20 coups de langue jusqu'à ce qu'il s'arrête ou jusqu'à la fin de la session.

Dans ces conditions expérimentales, les animaux de contrôle acceptent, en moyenne, 3 à 6 chocs. Le nombre de chocs obtenus avec les animaux traités est noté, et on compare ce nombre avec celui des animaux témoins par un test de Dunett. On détermine ainsi la DEM, dose efficace minimale, qui est la première dose augmentant de façon significative le nombre de chocs acceptés par un animal, par rapport aux témoins. Les DEM se situent entre 3 et 100 mg/kg par voie intrapéritonéale.

Action sur l'électrocorticogramme du rat curarisé ventilé.

L'activité sédative ou hypnotique des composés a été déterminée par l'observation de leur action sur l'électrocorticogramme du rat selon la méthode décrite par H. Depoortere, Rev. E.E.G. Neurophysiol., 10, 3, 207–214 (1980) et par H. Depoortere et M. Decobert, J. Pharmacol. (Paris), 14, 2, 195–265 (1983).

Les produits à étudier ont été administrés par voie intrapéritonéale aux doses croissantes de 1 à 30 mg/kg. Ils induisent des tracés de sommeil à partir de doses allant de 3 à 30 mg/kg.

Effets sur la durée du «sommeil» induit par le hydroxy-4 butyrate de sodium.

Cette action a été déterminée par l'influence d'un composé sur la durée du «sommeil» induit par le hydroxy-4 butyrate de sodium (GHB) chez le rat curarisé.

Les animaux utilisés sont des rats mâles de souche Charles River de 200 ± 20 g. Les animaux, curarisés par l'alloférine à raison de 1 mg/kg par voie i.p., sont placés sous respiration artificielle à l'aide d'un masque appliqué sur le museau (fréquence respiratoire = 50/minute; volume respiratoire = 14 ml).

L'œsophage est préalablement ligaturé afin d'éviter l'entrée de l'air dans l'estomac.

Des électrodes corticales front-pariétales et occipitales permettent l'enregistrement de l'activité électrocorticographique sur un polygraphe Grass modèle 79 P à la vitesse de 6 mm/sec.

La préparation de l'animal est effectuée sous anesthésie locale (xylocaïne à 2%). Les rats sont maintenus tout au long de l'expérience à température constante (37,5 C°). Dix minutes après la fin de la préparation du rat, une dose de 200 mg/kg de hydroxy-4 butyrate de sodium est injectée par voie intraveineuse au niveau de la queue.

Une dose de 10 mg/kg du composé à étudier est administrée par voie intrapéritonéale 3 minutes après l'administration du hydroxy-4 butyrate de sodium.

L'évaluation des tracés s'effectue par périodes de 15 minutes durant 75 minutes après l'injection de GHB. Durant cette période d'analyse, la durée totale du «sommeil» est déterminée. Une série de 15 témoins permet de préciser la durée du «sommeil GHB».

L'analyse statistique des résultats est réalisée à l'aide du test «U» de Mann-Whitney.

Certains composés réduisent les effets du GHB (jusqu'à 24% de diminution de la durée du sommeil à la dose de 10 mg/kg), tandis que d'autres potentialisent ces effets (jusqu'à 18% d'augmentation de la durée du sommeil à la dose de 10 mg/kg, ou 30% à la dose de 30 mg/kg). On constate également que les effets peuvent être opposés selon que les composés sont administrés à fortes doses ou à faibles doses.

Les résultats de ces différents tests montrent que les composés de l'invention possèdent des propriétés anxiolytiques, inductrices de sommeil, hypnotiques et anticonvulsivantes; les composés de l'invention sont utiles pour le traitement des états d'anxiété, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens et pour le traitement des encéphalopathies métaboliques.

Les composés de l'invention peuvent être présentés sous toute forme appropriée pour l'administration par voie orale ou parentérale, par exemple sous forme de comprimés, de dragées, de gélules, de solutions buvables ou injectables etc, en association avec tout excipient approprié. La posologie quotidienne peut aller de 1 à 100 mg.

**Revendications pour les états contractants: BE, CH, DE, FR, GB, IT, LI, LU, NL, SE**

1. Composés répondant à la formule générale I

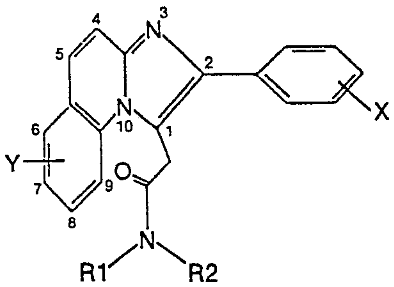

(I)

dans laquelle

X représente l'hydrogène, un halogène ou un groupe méthyle, méthoxy, méthylthio ou méthylsulfonyle,

Y représente l'hydrogène, un halogène ou un groupe méthyle en position 6, 7 ou 8, et

R1 et R2, pris séparément, représentent chacun l'hydrogène ou un groupe méthyle ou éthyle, ou bien

R1 et R2 forment ensemble une chaîne tétraméthylène, pentaméthylène, méthyl-3 aza-3 pentaméthylène, éthoxycarbonyl-3 aza-3 pentaméthylène, ou oxa-3 pentaméthylène, ainsi que leurs sels d'addition acceptables en pharmacologie.

2. Procédé de préparation des composés selon la revendication 1, caractérisé en ce que

. soit on soumet la quinoline de formule II

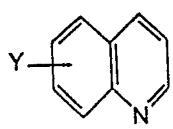

(II)

à l'action d'une α-bromo-acétophénone portant le substituant X défini à la revendication 1, pour obtenir un composé ionique de formule III

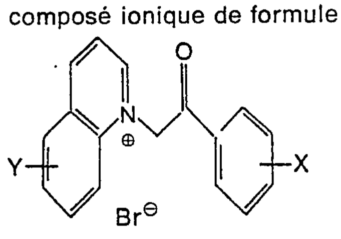

(III)

que l'on cyclise à chaud en présence d'acétate d'ammonium et de chlorure ferrique,

. soit on soumet l'amino-2 quinoline de formule IV

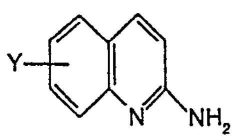

(IV)

à l'action d'une α-bromo-acétophénone portant le substituant X défini à la revendication 1, obtenant ainsi le composé de formule V

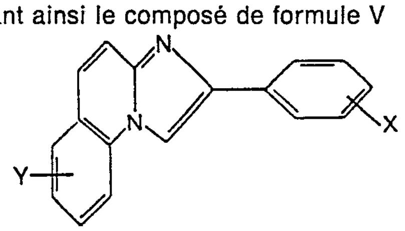

(V)

que l'on soumet à une formylation pour obtenir l'aldéhyde de formule VI

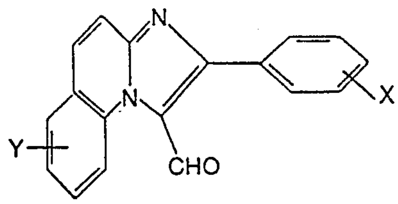

(VI)

puis on réduit ce composé en un alcool de formule VII

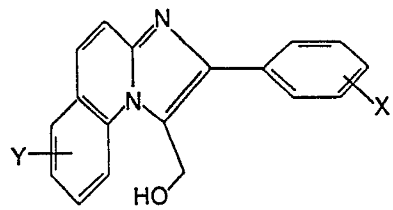

(VII)

que l'on transforme en nitrile de formule VIII

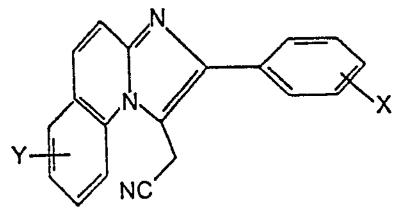

(VIII)

que l'on hydrolyse pour obtenir l'acide de formule IX

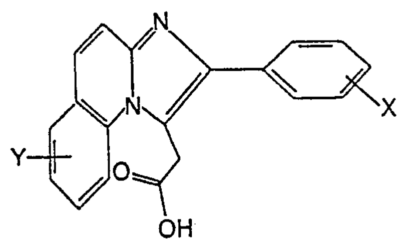

(IX)

et finalement on amidifie ce dernier en le faisant réagir avec une amine de formule R1-NH-R2 dans laquelle R1 et R2 sont tels que définis à la revendication 1.

3. Procédé selon la revendication 2, caractérisé en ce qu'on prépare l'amide de formule I dans laquelle R1 et R2 représentent chacun l'hydrogène par hydrolyse du nitrile de formule VIII.

4. Procédé selon l'une des revendications 2 et 3, caractérisé en ce qu'on prépare l'amide de formule I dans laquelle R1 et R2 représentent chacun un groupe méthyle par méthylation directe de l'amide de formule I dans laquelle R1 et R2 représentent chacun l'hydrogène.

5. Médicament, caractérisé en ce qu'il est constitué d'un composé selon la revendication 1.

6. Composition pharmaceutique, caractérisée en ce qu'elle contient un composé selon la revendication 1, en association avec un excipient approprié.

**Revendications pour l'état contractant: AT**

1. Procédé de préparation de composés répondant à la formule générale I

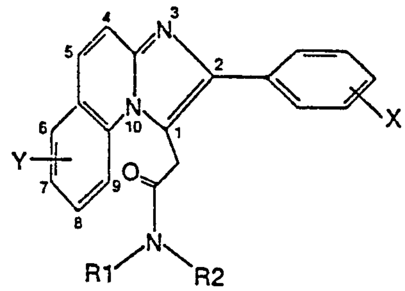

(I)

dans laquelle

X représente l'hydrogène, un halogène ou un groupe méthyle, méthoxy, méthylthio ou méthylsulfonyle,

Y représente l'hydrogène, un halogène ou un groupe méthyle en position 6, 7 ou 8, et

R1 et R2, pris séparément, représentent chacun l'hydrogène ou un groupe méthyle ou éthyle, ou bien

R1 et R2 forment ensemble une chaîne tétraméthylène, pentaméthylène, méthyl-3 aza-3 pentaméthylène, éthoxycarbonyl-3 aza-3 pentaméthylène, ou oxa-3 pentaméthylène,

procédé caractérisé en ce que

. soit on soumet la quinoline de formule II

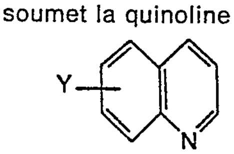

(II)

à l'action d'une α-bromo-acétophénone portant le substituant X défini ci-dessus, pour obtenir un composé ionique de formule III

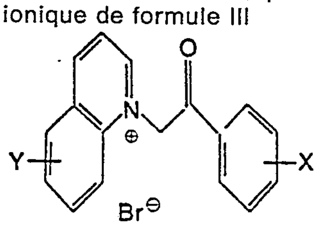

(III)

que l'on cyclise à chaud en présence d'acétate d'ammonium et de chlorure ferrique,

. soit on soumet l'amino-2 quinoline de formule IV

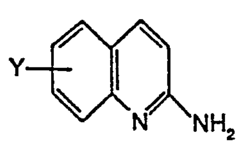

(IV)

à l'action d'une α-bromo-acétophénone portant le substituant X défini ci-dessus,

obtenant ainsi le composé de formule V

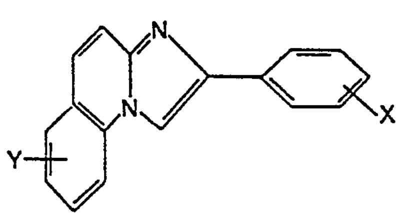

(V)

que l'on soumet à une formylation pour obtenir l'aldéhyde de formule VI

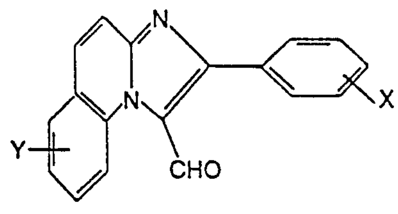

(VI)

puis on réduit ce composé en un alcool de formule VII

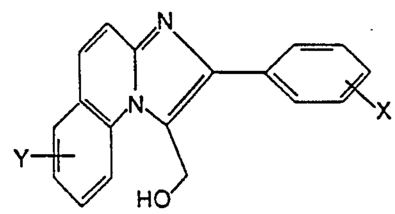

(VII)

que l'on transforme en nitrile de formule VIII

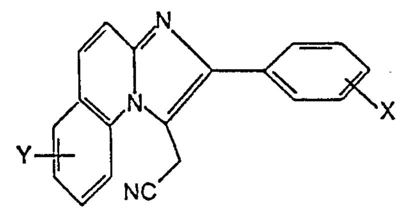

(VIII)

que l'on hydrolyse pour obtenir l'acide de formule IX

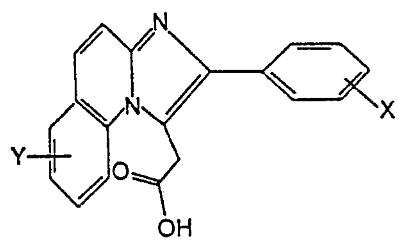

(IX)

et finalement on amidifie ce dernier en le faisant réagir avec une amine de formule R1-NH-R2 dans laquelle R1 et R2 sont tels que définis ci-dessus.

2. Procédé selon la revendication 1, caractérisé en ce qu'on prépare l'amide de formule I dans laquelle R1 et R2 représentent chacun l'hydrogène par hydrolyse du nitrile de formule VIII.

3. Procédé selon l'une des revendications 1 et 2, caractérisé en ce qu'on prépare l'amide de formule I dans laquelle R1 et R2 représentent chacun un groupe méthyle par méthylation directe de l'amide de formule I dans laquelle R1 et R2 représentent chacun l'hydrogène.

**Claims for the contracting states: BE, CH, DE, FR, GB, IT, LI, LU, NL, SE**

1. Compounds corresponding to the general formula I

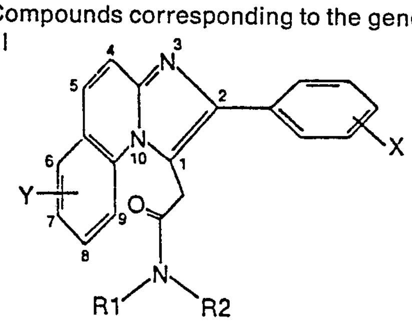

(I)

in which

X denotes hydrogen, a halogen or a methyl, methoxy, methylthio or methylsulphonyl group,

Y denotes hydrogen, a halogen or a methyl group at the 6-, 7- or 8-position, and

R1 and R2, taken separately, each denote hydrogen or a methyl or ethyl group, or alternatively

R1 and R2 together form a tetramethylene, pentamethylene, 3-methyl-3-azapentamethylene, 3-ethoxycarbonyl-3-azapentamethylene or 3-oxapentamethylene chain,

as well as their pharmacologically acceptable addition salts.

2. Process for preparing the compounds according to Claim 1, characterized in that

. either the quinoline of formula II

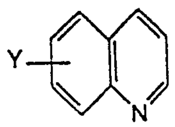

(II)

is subjected to the action of an α-bromoacetophenone bearing the substituent X defined in Claim 1, to obtain an ionic compound of formula III

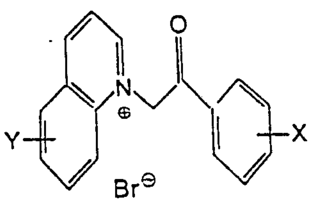

(III)

which is cyclized in the heated state in the presence of ammonium acetate and ferric chloride,

. or the 2-aminoquinoline of formula IV

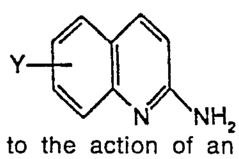

(IV)

is subjected to the action of an α-bromoacetophenone bearing the substituent X defined in Claim 1,

thereby obtaining the compound of formula V

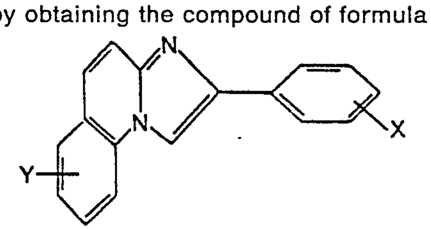

(V)

which is subjected to a formylation to obtain the aldehyde of formula VI

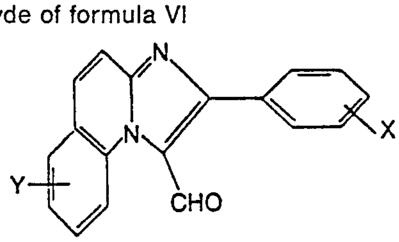

(VI)

this compound is then reduced to an alcohol of formula VII

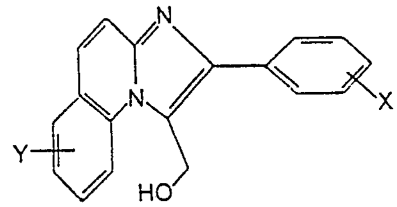

(VII)

which is converted to a nitrile of formula VIII

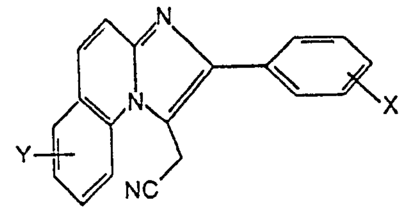

(VIII)

which is hydrolysed to obtain the acid of formula IX

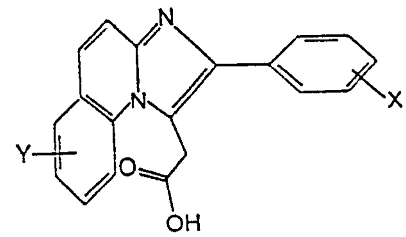

(IX)

and the latter is finally amidated by reacting it with an amine of formula R1-NH-R2 in which R1 and R2 are as defined in Claim 1.

3. Process according to Claim 2, characterized in that the amide of formula I in which R1 and R2 each denote hydrogen is prepared by hydrolysis of the nitrile of formula VIII.

4. Process according to one of Claims 2 and 3, characterized in that the amide of formula I in which R1 and R2 each denote a methyl group is prepared by direct methylation of the amide of formula I in which R1 and R2 each denote hydrogen.

5. Medicinal product, characterized in that it consists of a compound according to Claim 1.

6. Pharmaceutical composition, characterized in that it contains a compound according to Claim 1, in combination with a suitable excipient.

**Claims for the contracting state: AT**

1. Process for preparing compounds corresponding to the general formula I

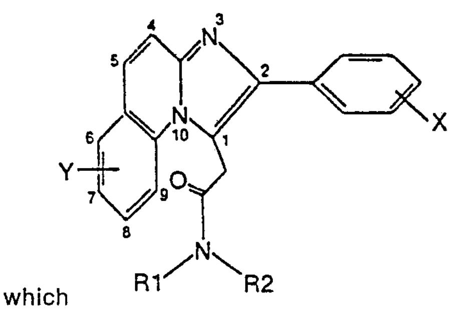

(I)

in which

X denotes hydrogen, a halogen or a methyl, methoxy, methylthio or methylsulphonyl group,

Y denotes hydrogen, a halogen or a methyl group at the 6-, 7- or 8-position, and

R1 and R2, taken separately, each denote hydrogen or a methyl or ethyl group, or alternatively

R1 and R2 together form a tetramethylene, pentamethylene, 3-methyl-3-azapentamethylene, 3-ethoxycarbonyl-3-azapentamethylene or 3-oxapentamethylene chain,

which process is characterized in that

. either the quinoline of formula II

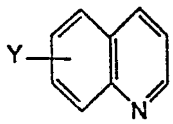

(II)

is subjected to the action of an α-bromoacetophenone bearing the substituent X defined above, to obtain an ionic compound of formula III

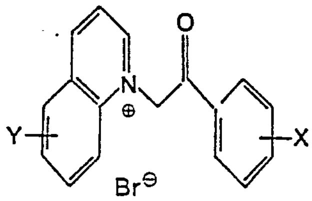

(III)

which is cyclized in the heated state in the presence of ammonium acetate and ferric chloride,

. or the 2-aminoquinoline of formula IV

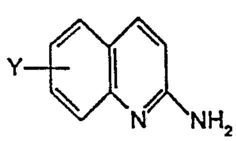

(IV)

is subjected to the action of an α-bromoacetophenone bearing the substituent X defined above, thereby obtaining the compound of formula V

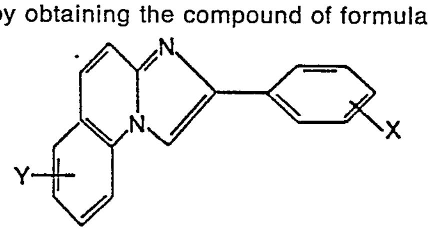

(V)

which is subjected to a formylation to obtain the aldehyde of formula VI

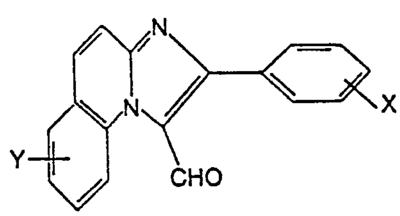

(VI)

this compound is then reduced to an alcohol of formula VII

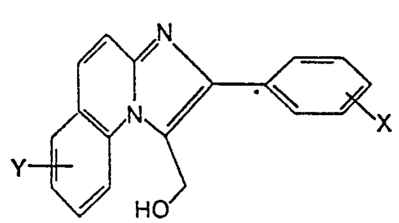

(VII)

which is converted to a nitrile of formula VIII

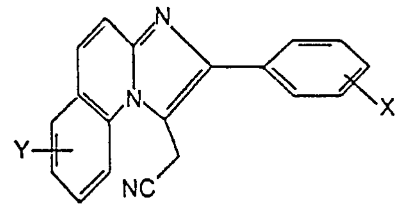

(VIII)

which is hydrolysed to obtain the acid of formula IX

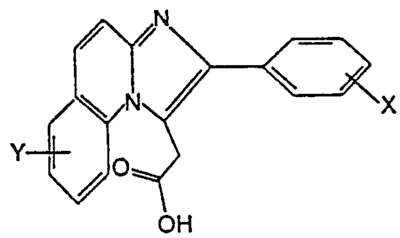

(IX)

and the latter is finally amidated by reacting it with an amine of formula R1-NH-R2 in which R1 and R2 are as defined above.

2. Process according to Claim 1, characterized in that the amide of formula I in which R1 and R2 each denote hydrogen is prepared by hydrolysis of the nitrile of formula VIII.

3. Process according to one of Claims 1 and 2, characterized in that the amide of formula I in which R1 and R2 each denote a methyl group is prepared by direct methylation of the amide of formula I in which R1 and R2 each denote hydrogen.

**Patentansprüche für die Vertragsstaaten BE, CH, DE, FR, GB, IT, LI, LU, NL, SE**

1. Verbindungen der allgemeinen Formel I

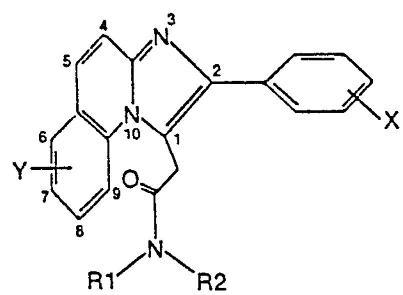

(I)

wobei

X Wasserstoff, ein Halogen oder eine Methyl-, Methoxy-, Methylthio- oder Methylsulfonylgruppe,

Y Wasserstoff, ein Halogen oder eine Methylgruppe in Stellung 6, 7 oder 8, und

R1 und R2 getrennt jeweils Wasserstoff oder eine Methyl- oder Äthylgruppe, oder aber

R1 und R2 gemeinsam eine Tetramethylen-, Pentamethylen, 3-Methyl-3-aza-pentamethylen-, 3-Äthoxycarbonyl-3-aza-pentamethylen oder 3-Oxa-pentamethylen-Kette bedeuten,

sowie ihre in der Pharmakologie verwendbaren Additionssalze.

2. Verfahren zur Herstellung von Verbindungen nach Anspruch 1, dadurch gekennzeichnet, dass

. entweder ein Chinolin der Formel II

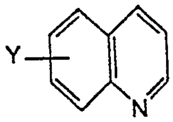

(II)

der Einwirkung eines α-Brom-acetophenons unterworfen wird, das den im Anspruch 1 definierten Substituenten X trägt, um eine ionische Verbindung der Formel III zu erhalten

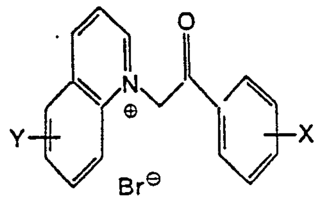

(III)

welche in der Hitze in Anwesenheit von Ammoniumacetat und Eisen(III)chlorid zyklisiert wird,
. oder ein 2-Aminochinolin der Formel IV

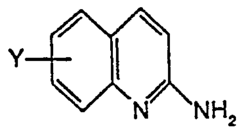

(IV)

der Einwirkung eines α-Brom-acetophenons unterworfen wird, das den im Anspruch 1 definierten Substituenten X trägt, um so die Verbindung der Formel V zu erhalten

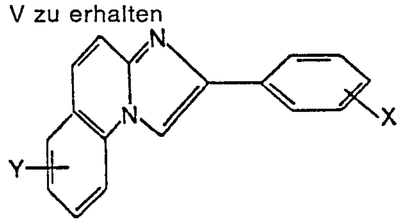

(V)

welche einer Formylierung unterworfen wird, um den Aldehyd nach Formel VI zu erhalten

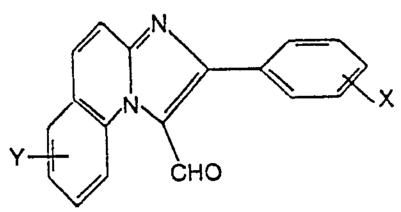

(VI)

der hierauf zu einem Alkohol der Formel VII reduziert wird

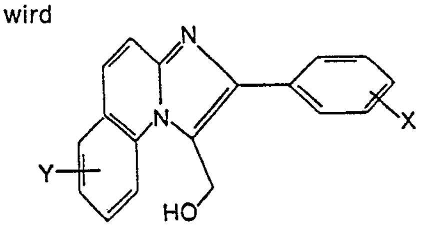

(VII)

welcher in ein Nitril der Formel VIII umgewandelt wird

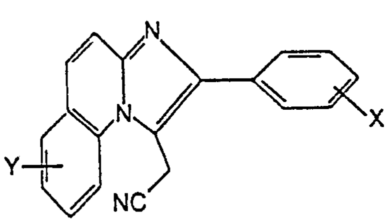

(VIII)

das zur Säure der Formel IX hydrolisiert wird

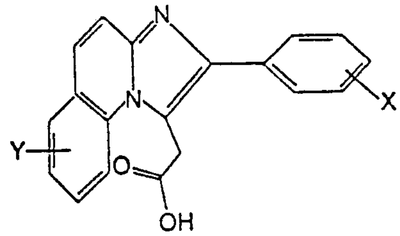

(IX)

die schließlich durch Reaktion mit einem Amin der Formel R1-NH-R2, wobei R1 und R2 wie in Anspruch 1 definiert sind, amidiert wird.

3. Verfahren nach Anspruch 2, dadurch gekennzeichnet, daß das Amid der Formel I, bei welchem R1 und R2 jeweils Wasserstoff bedeuten, durch Hydrolyse des Nitrils der Formel VIII hergestellt wird.

4. Verfahren nach einem der Ansprüche 2 und 3, dadurch gekennzeichnet, daß das Amid nach Formel I bei dem R1 und R2 jeweils eine Methylgruppe darstellen, hergestellt wird durch direkte Methylierung des Amids nach Formel I, bei dem R1 und R2 jeweils Wasserstoff darstellen.

5. Arzneimittel, dadurch gekennzeichnet, daß es aus einer Verbindung nach Anspruch 1 besteht.

6. Pharmazeutische Zubereitung, dadurch gekennzeichnet, daß sie eine Verbindung nach Anspruch 1 in Verbindung mit einem geeigneten Exzipiens enthält.

**Patentansprüche für den Vertragsstaat AT**

1. Verfahren zur Herstellung von Verbindungen der allgemeinen Formel I

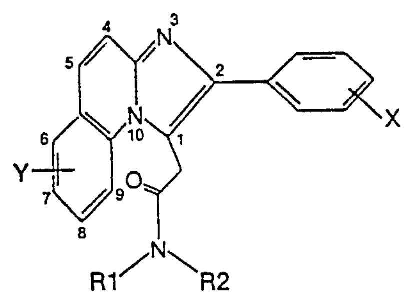

(I)

wobei

X Wasserstoff, ein Halogen oder eine Methyl-, Methoxy-, Methylthio- oder Methylsulfonylgruppe,

Y Wasserstoff, ein Halogen oder eine Methylgruppe in Stellung 6, 7 oder 8, und

R1 und R2 getrennt jeweils Wasserstoff oder eine Methyl- oder Äthylgruppe, oder aber

R1 und R2 gemeinsam eine Tetramethylen-, Pentamethylen, 3-Methyl-3-aza-pentamethylen-, 3-Äthoxycarbonyl-3-aza-pentamethylen oder 3-Oxa-pentamethylen-Kette

bedeuten, dadurch gekennzeichnet, daß
. entweder ein Chinolin der Formel II

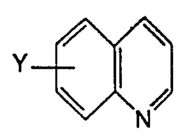

(II)

der Einwirkung eines α-Brom-acetophenons unterworfen wird, das den oben definierten Substituenten X trägt, um eine ionische Verbindung der Formel III zu erhalten

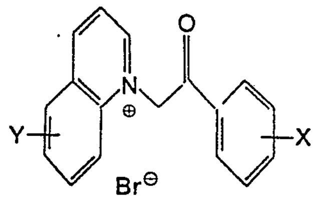

(III)

welche in der Hitze in Anwesenheit von Ammoniumacetat und Eisen(III)chlorid zyklisiert wird, . oder ein 2-Aminochinolin der Formel IV

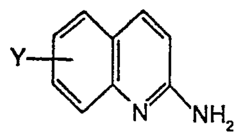

(IV)

der Einwirkung eines α-Brom-acetophenons unterworfen wird, das den oben definierten Substituenten X trägt, um so die Verbindung der Formel V zu erhalten

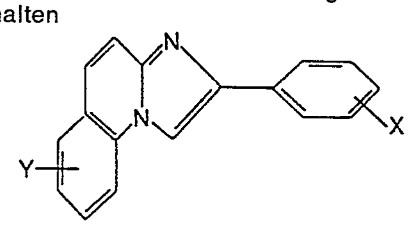

(V)

welche einer Formylierung unterworfen wird, um den Aldehyd nach Formel VI zu erhalten

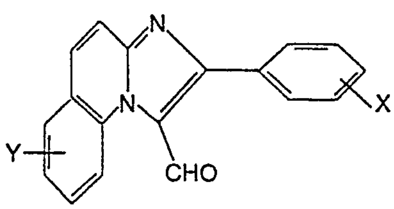

(VI)

der hierauf zu einem Alkohol der Formel VII reduziert wird

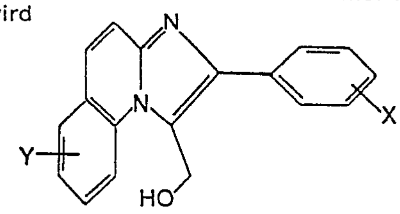

(VII)

welcher in ein Nitril der Formel VIII umgewandelt wird

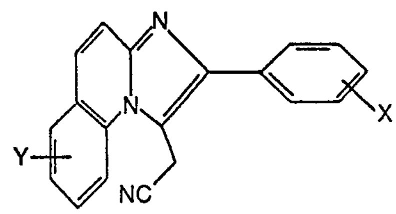

(VIII)

das zur Säure der Formel IX hydrolisiert wird

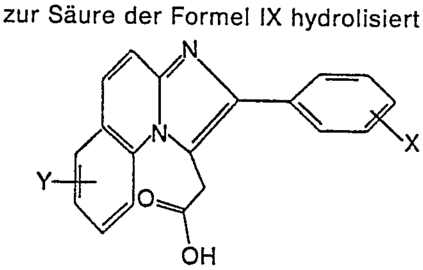

(IX)

die schließlich durch Reaktion mit einem Amin der Formel R1-NH-R2, wobei R1 und R2 wie oben definiert sind, amidiert wird.

2. Verfahren nach Anspruch 1, dadurch gekennzeichnet, daß das Amid der Formel I, bei welchem R1 und R2 jeweils Wasserstoff bedeuten, durch Hydrolyse des Nitrils der Formel VIII hergestellt wird.

3. Verfahren nach einem der Ansprüche 1 und 2, dadurch gekennzeichnet, daß das Amid nach Formel I bei dem R1 und R2 jeweils eine Methylgruppe darstellen, hergestellt wird durch direkte Methylierung des Amids nach Formel I, bei dem R1 und R2 jeweils Wasserstoff darstellen.